(19) Europäisches Patentamt / European Patent Office / Office européen des brevets

(11) **EP 4 192 558 B1**

(12) **EUROPEAN PATENT SPECIFICATION**

(45) Date of publication and mention
of the grant of the patent:
**24.12.2025 Bulletin 2025/52**

(51) International Patent Classification (IPC):
*A61M 16/12* (2006.01)      *A61M 16/00* (2006.01)
*A61M 16/16* (2006.01)      *A61M 16/10* (2006.01)

(21) Application number: **21762102.8**

(22) Date of filing: **30.07.2021**

(52) Cooperative Patent Classification (CPC):
**A61M 16/125; A61M 16/021;** A61M 16/0066;
A61M 16/161; A61M 2016/0039; A61M 2016/1025;
A61M 2202/0208; A61M 2205/18; A61M 2205/3355;
A61M 2205/3368; A61M 2205/505; A61M 2205/581;
A61M 2205/583; A61M 2205/584; A61M 2230/04;
(Cont.)

(86) International application number:
**PCT/IB2021/056980**

(87) International publication number:
**WO 2022/029582 (10.02.2022 Gazette 2022/06)**

(54) **DEVICE FOR PULMONARY VENTILATION**

VORRICHTUNG ZUR PULMONALEN BEATMUNG

DISPOSITIF DE VENTILATION PULMONAIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **07.08.2020 IT 202000019828**

(43) Date of publication of application:
**14.06.2023 Bulletin 2023/24**

(73) Proprietor: **IBD Italian Biomedical Devices S.r.l.
46043 Castiglione delle Stiviere (IT)**

(72) Inventors:
• **VISOTTI, Andrea
46043 Castiglione delle Stiviere (IT)**
• **GHIDINI, Corrado
46043 Castiglione delle Stiviere (IT)**
• **DRUDI, Debora
46043 Castiglione delle Stiviere (IT)**
• **PERAZZINI, Claudia
46043 Castiglione delle Stiviere (IT)**

(74) Representative: **Zoli, Filippo
Brunacci & Partners S.r.l.
Via Pietro Giardini, 625
41125 Modena (IT)**

(56) References cited:
WO-A1-03/064009          WO-A1-2013/038342
WO-A1-2019/070136        US-A1- 2007 227 360
US-A1- 2015 144 136      US-A1- 2015 250 962

EP 4 192 558 B1

(52) Cooperative Patent Classification (CPC): (Cont.)
A61M 2230/205; A61M 2230/42

**Description**

Technical Field

The present invention relates to a device for pulmonary ventilation, particularly for high-flow oxygen therapy and sub-intensive therapy.

Background Art

As is well known, pulmonary ventilation, also known as artificial ventilation or mechanical ventilation, replaces or supports the activity of the inspiratory muscles by ensuring an adequate volume of gas to the lungs.

Pulmonary ventilators thus replace, in whole or in part, the mechanical functions of the respiratory system when the latter becomes incapable of performing its task alone, due to disease, trauma, congenital defects or medication.

The ventilator is capable of insufflating a certain mixture of gases, generally air and oxygen, into the lungs and then allowing them to exhale, at a known frequency and with appropriate pressure.

The pulmonary ventilators known today generally comprise a box-shaped structure inside which a circuit is housed for mixing air with oxygen and its insufflation under pressure to the patient, and an electronic control unit adapted to control the circuit mentioned above, to detect a series of physical quantities such as pressure, gas flow, amount of oxygen supplied, etc.... and to display these physical quantities on a special display screen.

In particular, the circuit for mixing air and oxygen comprises a plurality of hoses, generally made of polymeric material, connected to each other in a fluid-operated manner and connected to a plurality of auxiliary devices such as, e.g., pressure sensors, flow meters, solenoid valves or oxygen detectors.

Nevertheless, these ventilators of known type do have some drawbacks, due in particular to the complex assembly of the fluid-operated circuit.

The large number of components to be assembled and their overall dimensions affect not only the time and cost of manufacture, but also the size.

Known ventilators are, in fact, generally very bulky and specifically adapted to hospital use for the treatment of intubated patients, who are therefore unable to breathe spontaneously, and for intensive care.

These limitations, both in terms of cost and size, significantly affect their use on emergency vehicles, such as ambulances, and in the case of patient home care. Devices of the known type for pulmonary ventilation are disclosed by US 2007/227360 A1, WO 2013/038342 A1, WO 2019/070136 A1, WO 03/064009 A1, US2015250962A1.

US2015144136 A1 discloses a device for pulmonary ventilation having a monolithic body comprising two shelves locked together with each other and inside which two breathing gases are mixed.

Description of the Invention

The main aim of the present invention is to provide a device for pulmonary ventilation which allows overcoming the drawbacks of the ventilators known to date, and in particular, which allows greatly reducing the assembly time and overall manufacturing costs.

Within this aim, one object of the present invention is to provide a device for pulmonary ventilation which is more flexible to use than the devices of known type and which, therefore, may be effectively used not only in the hospital sector, but also on the emergency vehicles and in the case of patient home care.

Another object of the present invention is to reduce noise to a minimum, as well as load losses and losses due to assemblies.

Another object of the present invention is to provide for a device for pulmonary ventilation which allows overcoming the aforementioned drawbacks of the prior art within a simple, rational, easy and efficient to use as well as affordable solution.

The aforementioned objects are achieved by the present device for pulmonary ventilation according to claim 1.

Brief Description of the Drawings

Other characteristics and advantages of the present invention will be more apparent from the description of a preferred, but not exclusive embodiment of a device for pulmonary ventilation, illustrated by way of an indicative, yet nonlimiting example, in the attached tables of drawings wherein:

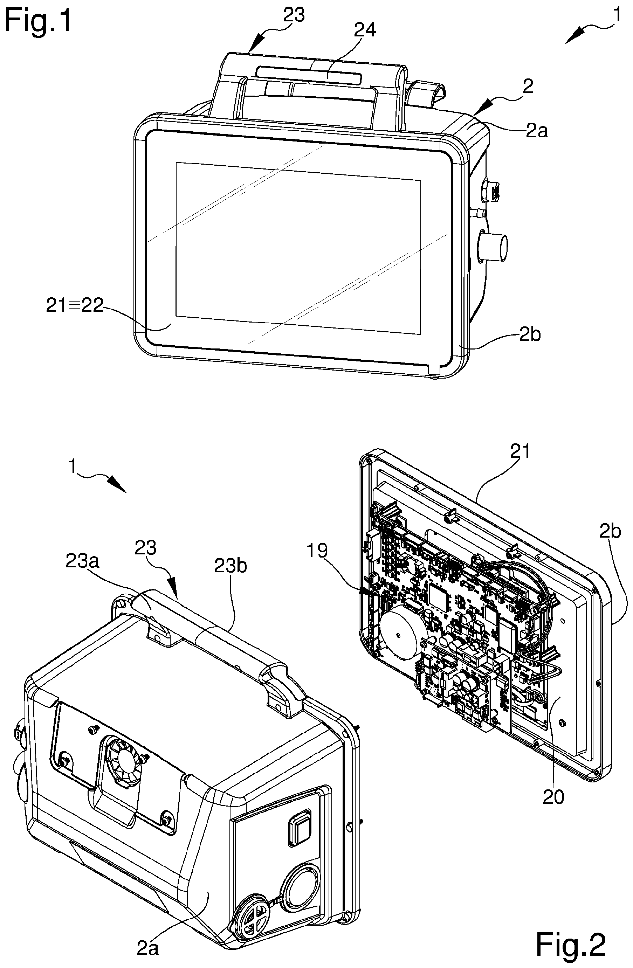

    Figure 1 is an axonometric view of a device for pulmonary ventilation according to the invention;
    Figure 2 is an exploded view of the box-shaped body of the device in Figure 1;
    Figure 3 is an exploded view of a part of the device in Figure 1;
    Figure 4 is a cross-sectional view of the device in Figure 1;

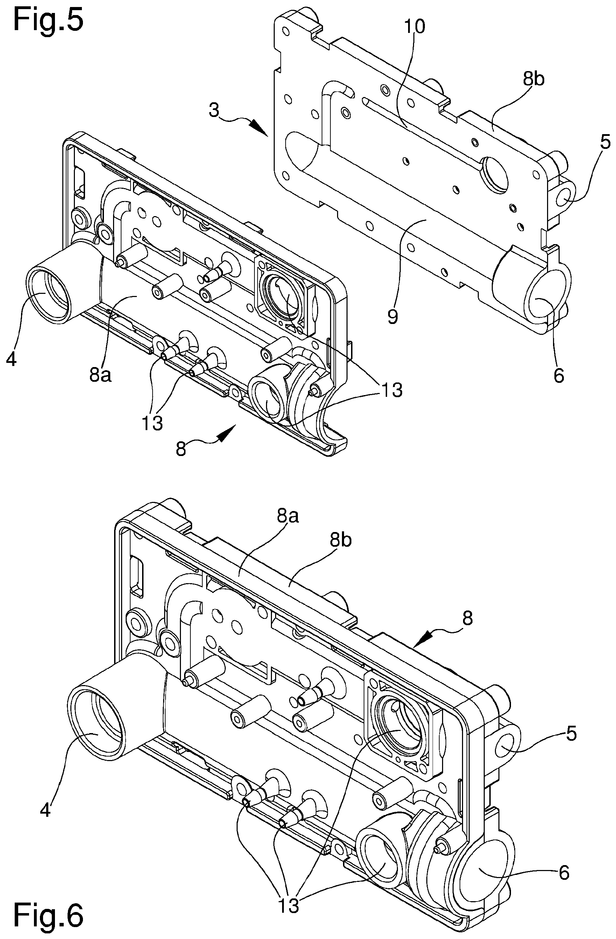

    Figure 5 is an exploded view of the rigid monolithic body of the device in Figure 1;
    Figure 6 is an axonometric view of the assembled rigid monolithic body in Figure 5;

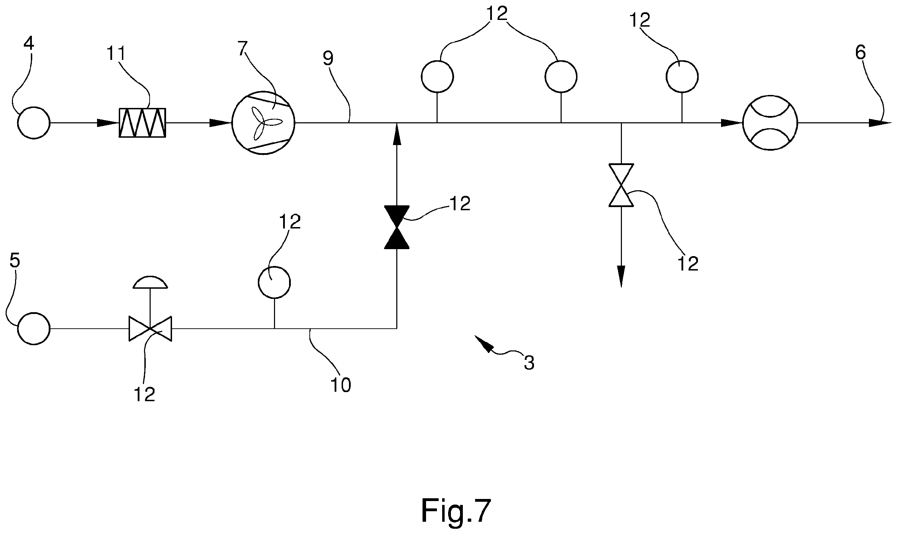

    Figure 7 is a schematic representation of the circuit of a device according to the device.

Embodiments of the Invention

With particular reference to these illustrations, reference numeral 1 globally indicates a device for pulmonary ventilation.

The device 1 is particularly adapted to be used in high-flow oxygen therapy and sub-intensive care ventilation.

The device 1 comprises a box-shaped body 2 and a fluid-operated circuit 3 accommodated inside it.

The circuit 3 comprises at least one inlet port 4 for the air, at least one inlet mouth 5 for the oxygen, at least one outlet port 6 for a mixture of air and oxygen, and a device to generate a positive pressure 7.

According to the invention, the circuit 3 comprises a rigid monolithic body 8 in turn comprising at least one main channel 9, which defines the inlet port 4 for the air and the outlet port 6 for a mixture, and at least one secondary channel 10, which defines the inlet mouth 5 for the oxygen and which communicates with the main channel 9. The secondary channel 10 is connectable, by means of its inlet mouth 5, to a source of oxygen under pressure, not shown in the illustrations, and thus allows oxygen under pressure to be supplied along the main channel 9 in order to mix it with the air passing through the main channel itself.

Preferably, the rigid monolithic body 8 comprises a first half-shell 8a and a second half-shell 8b locked together with each other.

The first half-shell 8a and the second half-shell 8b can be associated with each other by means of threaded members, in which case at least one sealing element is interposed between them, or they can be made mutually integral by welding or adhesive means.

More particularly, the inlet port 4 for the air and the outlet port 6 for a mixture are angularly staggered to each other by an angle comprised between 80° and 100°, preferably equal to about 90°. By this definition it is meant that the lying planes defined by the inlet port 4 and by the outlet port 6 are angularly staggered to each other.

In this embodiment, the main channel 9 therefore comprises at least one curved section, identified in the illustration by reference numeral 9a, having a radius of curvature comprised between 9 mm and 11 mm, preferably between 9.5 mm and 10.5 mm.

This conformation of the main channel 9 allows preventing turbulent states of the gas flowing through it and which can generate a background noise.

Alternative embodiments cannot however be ruled out wherein the inlet port 4 for the air and the outlet port 6 for the mixture are arranged substantially parallel to each other.

According to the invention, the device to generate a positive pressure 7 is connected to the rigid monolithic body 8 in a fluid-operated manner.

Conveniently, in the embodiment shown in the illustrations, the device to generate a positive pressure 7 is directly associated, in a removable manner, with the inlet port 4 defined by the main channel 9.

The device to generate a positive pressure 7 is, e.g., snap-fitted through the inlet port 4. Alternative forms of connection, known to the technician in the sector, to the rigid monolithic body 8 cannot however be ruled out.

In an alternative embodiment, not shown in the illustrations, the circuit 3 also comprises at least one additional device to generate a positive pressure which is associated in a removable manner with the inlet mouth 5 for the oxygen.

Advantageously, the device to generate a positive pressure 7 is of the type of a turbine provided with an electric motor and adapted to receive an inlet gas, air in the embodiment described herein, in order to increase the outlet pressure thereof.

More particularly, the device to generate a positive pressure 7 is provided with an air inlet opening, not visible in detail in the illustrations, at which a filtering element 11 is arranged.

The device 1 comprises a plurality of auxiliary devices 12 operatively connected to the circuit 3 and accommodated inside the box-shaped body 2.

Advantageously, the rigid monolithic body 8 comprises a plurality of predefined openings 13 with which one or more auxiliary devices 12 are directly associated. In other words, the auxiliary devices 12 are associated with the rigid monolithic body 8.

The auxiliary devices 12 can be associated with the predefined openings 13 in a removable manner, so as to allow the removal and maintenance thereof, or in an unmovable manner. More particularly, the predefined openings 13 communicate with at least one of either the main channel 9 or the secondary channel 10.

Conveniently, the auxiliary devices 12 are associated by sealing with the relevant predefined openings 13.

The auxiliary devices 12 are selected from the group comprising: flow meters, oxygen sensors, pressure sensors, pressure regulators, solenoid valves, temperature detectors and humidity detectors.

More in detail, the flow meter is adapted to detect the flow (flow rate) of the air and oxygen mixture flowing through the main channel 9.

The oxygen sensor is adapted to detect the oxygen fraction $FiO_2$ present in the air and oxygen mixture flowing through the main channel 9.

The pressure sensor is in turn adapted to detect the pressure of the air and oxygen mixture flowing through the main channel 9.

Preferably, the box-shaped body 2 comprises a first body 2a defining a housing seat 14 of the rigid monolithic body 8, provided with at least one access opening 15, and comprises at least one second body 2b associated in a removable manner with the first body 2a for the closure of the access opening 15.

The first body 2a and the second body 2b are associated with each other by means of, e.g., a plurality of threaded members (not shown in detail in the illustrations) which fit inside relevant seats obtained on the first body 2a, passing through the second body 2b.

Advantageously, the rigid monolithic body 8 is associated in a removable manner with the first body 2a and rigidly supported by the latter.

In more detail, removable connection means 16 are provided of the monolithic body 8 to the first body 2a.

In the embodiment shown in the illustrations, the connection means 16 comprise a plurality of threaded members fitting inside the relevant seats 17 obtained on the first body 2a, passing through the rigid monolithic body 8.

Alternative embodiments cannot however be ruled out wherein, e.g., the connection means 16 are of the interlocking type.

By operating on the connection means 16, it is therefore possible to associate or remove the rigid monolithic body 8 to/from the first body 2a.

Appropriately, the first body 2a also comprises one or more accesses 18 adapted to put the housing seat 14 in communication with the outside to allow, e.g., the entry of air into the device to generate a positive pressure 7 and the outflow of the mixture of air and oxygen towards the outside.

The device 1 also comprises at least one electronic control unit 19 provided with a microprocessor and operatively connected to the device to generate a positive pressure 7 and to one or more auxiliary devices 12.

In particular, the electronic control unit 19 is configured to control the operation of the device to generate a positive pressure 7 and the microprocessor is configured to receive and process the data received from the auxiliary devices 12.

Conveniently, the second body 2b has an internal wall 20, facing the housing seat 14, and an external wall 21 accessible from the outside.

Advantageously, the electronic control unit 19 is associated with the internal wall 20 of the second body 2b and is electrically connected to both the device to generate a positive pressure 7 and to one or more auxiliary devices 12.

Preferably, the electronic control unit 19 is operatively connected to a graphical interface display 22 and the microprocessor is adapted to receive and process the data measured by the auxiliary devices 12 to make them appear at least partly on the display 22, in a graphical or numerical form.

Advantageously, the microprocessor is provided with means adapted to calculate the respiratory rate on the basis of the value of the flow of the air and oxygen mixture detected by the flow meter.

Adequately, the device 1 comprises at least one oximeter, not shown in the illustrations, operatively connected to the electronic control unit 19 and adapted to detect the blood saturation $SpO_2$ and the heart rate of the patient. The oximeter is positioned outside of the box-shaped body 2.

The microprocessor is also provided with means adapted to calculate an indicator of the effectiveness of the therapy on the patient, identified with the $RO_X$ index, on the basis of the values of the calculated heart rate, of the oxygen fraction $FiO_2$ detected by the oxygen sensor and of the blood saturation $SpO_2$ detected by the oximeter.

More in particular, the ROx index is calculated according to the formula:

$$RO_X = (SpO_2/FiO_2)/ \text{ heart rate.}$$

More in detail, the microprocessor is programmed to display on the display 22, in graphical and/or numerical form, the parameters selected from the group comprising: respiratory rate, oxygen fraction $FiO_2$, ROx index, heart rate, blood saturation SpO2.

Advantageously, the electronic control unit 19 also comprises a memory programmable with at least one value of reference of the ROx index and the microprocessor is configured to compare the calculated value of the ROx index with the preset value of reference.

The microprocessor is then configured to emit an alarm signal in the event of the calculated value of the ROx index being lower than the preset value of reference.

Conveniently, the values of the $RO_X$ index are shown on the display 22, in graphical and/or numerical form, with a first color, e.g. green, if they are higher than the preset value of reference and with a second color, e.g. red, if they are lower than the preset value of reference.

In this way, the medical staff may have an immediate and intuitive feedback of the pattern of the therapy on the patient.

In the preferred embodiment shown in the illustrations, the external wall 21 comprises the graphical interface display 22.

More in particular, the external wall 21 is composed of the display 22, which defines therefore a face of the device 1.

The display 22 is, e.g., of the touch-screen type.

The box-shaped body 2 also comprises at least one external handle 23 adapted to make its transport easier.

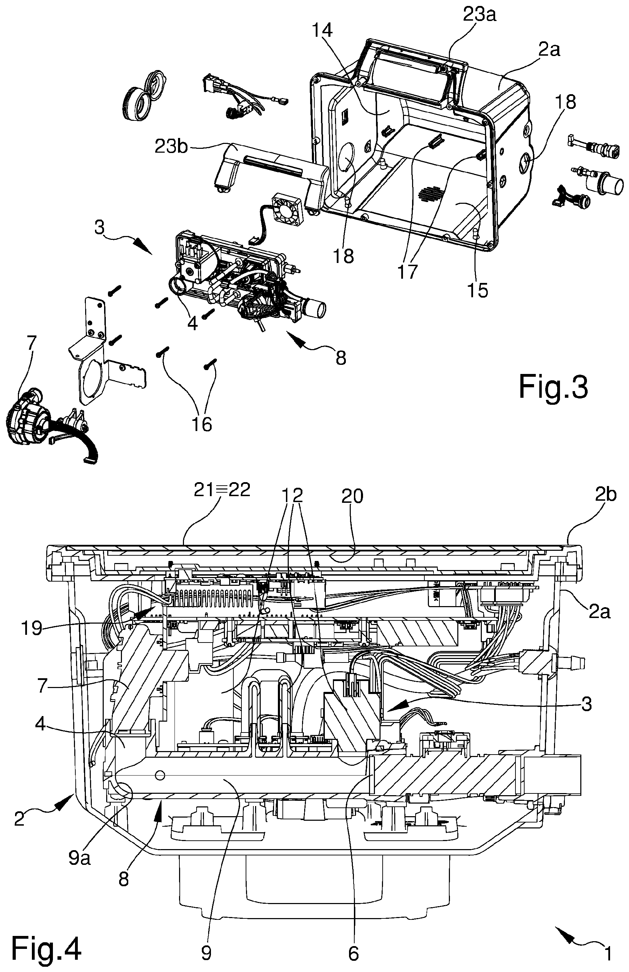

As can be seen from Figure 3, the handle 23 has at least a first portion 23a integrally defined with the first body 2a, inside which the electrical connections to the electronic control unit 19 are inserted, and a second portion 23b, adapted to close the first portion 23a and separate from both the first body 2a and the second body 2b.

Advantageously, the device 1 comprises acoustic and/or visual signaling means 24 operatively connected to the electronic control unit 19. The signaling means 24 are activated as a result of the detection of a malfunction by the electronic control unit 19, e.g. when one of the signals received by the auxiliary devices 12 exceeds a relevant preset threshold value.

In the embodiment shown in the illustrations, the signaling means 24 are integrally defined in the handle 23.

The assembly of the device according to the invention is described here below. Once all the above-described components making up the device 1 have been provided, the rigid monolithic body 8 is first assembled so as to define both the main channel 9 and the secondary channel 10.

This operation is performed by joining together the first half-shell 8a and the second half-shell 8b.

To the rigid monolithic body 8 thus obtained, both the device to generate a positive pressure 7 and the auxiliary devices 12 are fitted. Such fitting is performed by inserting the device to generate a positive pressure 7 inside the inlet port 4 and by associating the auxiliary devices 12 to the predefined openings 13, respectively.

The rigid monolithic body 8 is then positioned inside the housing seat 14 and is integrally associated with the first body 2a by means of the connection means 16.

At the same time, it is necessary to fit the electronic control unit 19 to the internal wall 20 of the second body 2b, after which the electrical connection is carried out of the electronic control unit itself to the device to generate a positive pressure 7 and to the auxiliary devices 12.

Finally, the first body 2a is locked together with the second body 2b, so as to close the access opening 15 and obtain the box-shaped body 2.

It has in practice been ascertained that the described invention achieves the intended objects and in particular the fact is underlined that the device for pulmonary ventilation, forming the subject of the present invention, thanks to the presence of the rigid monolithic body, is of simple realization and assembly and, consequently, inexpensive.

The realization of the main channel for the transit of air and of the secondary channel for the supply of oxygen inside the rigid monolithic body, and the presence on the latter of a plurality of predefined openings for connection to the auxiliary devices, does in fact significantly reduce the number of assembly operations that have to be performed, as well as their complexity.

Moreover, the use of the rigid monolithic body according to the present invention allows reducing to a minimum the overall dimensions of the components used and the size of the device thus obtained, facilitating the transportability and use thereof both on emergency vehicles and in the home care.

Last but not least, the use of an interface display as part of the box-shaped body which defines the external dimensions of the ventilation device allows saving on the use of material for the realization of the box-shaped body itself, thus optimizing costs and overall dimensions.

**Claims**

1. Device (1) for pulmonary ventilation, comprising:

> - a box-shaped body (2);
> - a circuit (3) accommodated inside said box-shaped body (2) and comprising at least: one inlet port (4) for the air, one inlet mouth (5) for the oxygen and one outlet port (6) for a mixture of air and oxygen, a device to generate a positive pressure (7);

> wherein said circuit (3) comprises a rigid monolithic body (8) comprising at least one main channel (9), which defines said inlet port (4) for the air and said outlet port (6) for a mixture, and at least one secondary channel (10), which defines said inlet mouth (5) for the oxygen and which communicates with said main channel (9), where said inlet mouth (5) is connectable to a source of oxygen under pressure and thus allows oxygen under pressure to be supplied along said main channel (9) in order to mix it with the air passing through the main channel itself and where said rigid monolithic body (8) defines said main channel (9) and defines at least partly said secondary channel (10),
> said rigid monolithic body (8) comprising a first half-shell (8a) and a second half-shell (8b) locked together with each other,
> wherein said circuit (3) comprises a plurality of auxiliary devices (12) selected from a group comprising at least one flow meter which is adapted to detect the flow rate of the air and oxygen mixture flowing through said main channel (9), an oxygen sensor adapted to detect the oxygen fraction ($FiO_2$) present in the air and oxygen mixture flowing through said main channel (9), a pressure sensor adapted to detect the pressure of the air and oxygen mixture flowing through said main channel (9),
> where said rigid monolithic body (8) comprises a plurality of predefined openings (13) and where said auxiliary devices (12) are associated with said predefined openings (13), said predefined openings (13) communicating with at least one of either said main channel (9) or said secondary channel (10), wherein said box-shaped body (2) comprises a first body (2a) defining a housing seat (14) of said rigid monolithic body (8) provided with at least one access opening (15) and comprises at least one second body (2b) associated in a removable manner with said first body (2a) for the closure of said access opening (15), and wherein said device comprises removable connection means (16) of said rigid monolithic body (8) to said first body (2a),
> wherein said device to generate a positive pressure (7) is connected in a fluid-operated manner to said rigid monolithic body (8),
> wherein said device to generate a positive pressure (7) is directly associated in a removable manner with said inlet port (4) for the air,

wherein the device (1) comprises at least one electronic control unit (19) provided with a microprocessor and operatively connected to said device to generate a positive pressure (7) and to said auxiliary devices (12) and

wherein said second body (2b) has an internal wall (20), facing said housing seat (14), and an external wall (21), facing outwards, where said electronic control unit (19) is associated with said internal wall (20),

wherein said electronic control unit (19) is operatively connected to a graphical interface display (22), said microprocessor being adapted to receive and process the signals detected at least by said auxiliary devices (12) to make them appear at least partly in graphical and/or numerical form on said graphical interface display (22),

and wherein said external wall (21) is composed of said graphical interface display (22), which defines a face of the device (1).

2. Device (1) according to claim 1, wherein said circuit (3) comprises at least one additional device for the generation of a positive pressure directly associated in a removable manner with said inlet mouth (5) for the oxygen.

3. Device (1) according to one or more of the preceding claims, wherein said auxiliary devices (12) are contained inside said box-shaped body (2).

4. Device (1) according to one or more of the preceding claims, wherein said inlet port (4) for the air and said outlet port (6) for a mixture are staggered to each other by an angle comprised between 80° and 100°.

5. Device (1) according to one or more of claims 1 to 3, wherein said inlet port (4) for the air and said outlet port (6) for a mixture are substantially parallel to each other.

6. Device (1) according to one or more of the preceding claims, wherein it comprises at least one oximeter positioned outside of said box-shaped body (2) and operatively connected to said electronic control unit (19), said oximeter being adapted to detect the blood saturation ($SpO_2$) and the heart rate of the patient.

7. Device (1) according to claim 6, wherein said microprocessor is provided with means adapted to calculate the respiratory rate on the basis of the value of the flow of the air and oxygen mixture detected by the flow meter and wherein said microprocessor is provided with means adapted to calculate an indicator of the effectiveness of the therapy on the patient ($RO_X$), on the basis of the values of the calculated heart rate, of the oxygen fraction ($FiO_2$) detected by said oxygen sensor and of the blood saturation ($SpO_2$) detected by said oximeter.

8. Device (1) according to claim 7, wherein said electronic control unit (19) comprises at least one memory programmable with at least one value of reference of the indicator of the effectiveness of the therapy on the patient ($RO_X$) and the microprocessor is configured to compare the calculated value of the indicator of the effectiveness of the therapy on the patient with said value of reference.

**Patentansprüche**

1. Vorrichtung (1) zur Lungenbeatmung, umfassend:

    - einen kastenförmigen Körper (2);
    - einen Kreislauf (3), der im Inneren des kastenförmigen Körpers (2) untergebracht ist und mindestens umfasst: eine Einlassöffnung (4) für die Luft, eine Einlassmündung (5) für den Sauerstoff und eine Auslassöffnung (6) für ein Gemisch aus Luft und Sauerstoff, eine Vorrichtung zur Erzeugung eines Überdrucks (7);
    wobei der Kreislauf (3) einen starren monolithischen Körper (8) umfasst, der mindestens einen Hauptkanal (9), der die Einlassöffnung (4) für die Luft und die Auslassöffnung (6) für ein Gemisch definiert, und mindestens einen Sekundärkanal (10) umfasst, der die Einlassmündung (5) für den Sauerstoff definiert und der mit dem Hauptkanal (9) in Verbindung steht, wobei die Einlassmündung (5) mit einer Quelle von unter Druck stehendem Sauerstoff verbindbar ist und somit ermöglicht, dass Sauerstoff unter Druck entlang des Hauptkanals (9) zugeführt wird, um ihn mit der Luft zu mischen, die durch den Hauptkanal selbst hindurchströmt, und wobei der starre monolithische Körper (8) den Hauptkanal (9) definiert und zumindest teilweise den Sekundärkanal (10) definiert, wobei der starre monolithische Körper (8) eine erste Halbschale (8a) und eine zweite Halbschale (8b) umfasst, die miteinander verriegelt sind,
    wobei der Kreislauf (3) eine Mehrzahl von Hilfsvorrichtungen (12) umfasst, die aus einer Gruppe ausgewählt sind, die mindestens ein Durchflussmessgerät umfasst, das dazu ausgebildet ist, die Durchflussrate des durch den Hauptkanal (9) strömenden Luft- und Sauerstoffgemisches zu erfassen, einen Sauerstoffsensor, der dazu ausgebildet ist, den in dem durch den Hauptkanal (9) strömenden Luft- und Sauerstoffgemisch vorhandenen Sauerstoffanteil

(FiO$_2$) zu erfassen, einen Drucksensor, der dazu ausgebildet ist, den Druck des durch den Hauptkanal (9) strömenden Luft- und Sauerstoffgemischs zu erfassen,

wobei der starre monolithische Körper (8) eine Mehrzahl von vordefinierten Öffnungen (13) umfasst und wobei die Hilfsvorrichtungen (12) mit den vordefinierten Öffnungen (13) verbunden sind, wobei die vordefinierten Öffnungen (13) mit dem Hauptkanal (9) und/oder dem Sekundärkanal (10) in Verbindung stehen, wobei der kastenförmige Körper (2) einen ersten Körper (2a) umfasst, der einen Gehäusesitz (14) des starren monolithischen Körpers (8) definiert, der mit mindestens einer Zugangsöffnung (15) versehen ist, und mindestens einen zweiten Körper (2b) umfasst, der auf lösbare Weise mit dem ersten Körper (2a) zum Verschließen der Zugangsöffnung (15) verbunden ist, und wobei die Vorrichtung entfernbare Mittel (16) zur Verbindung des starren monolithischen Körpers (8) mit dem ersten Körper (2a) umfasst,

wobei die Vorrichtung zur Erzeugung eines Überdrucks (7) mit dem starren monolithischen Körper (8) fluidbetrieben verbunden ist,

wobei die Vorrichtung zur Erzeugung eines Überdrucks (7) direkt mit der Einlassöffnung (4) für die Luft in einer lösbaren Weise verbunden ist,

wobei die Vorrichtung (1) mindestens eine elektronische Steuereinheit (19) umfasst, die mit einem Mikroprozessor versehen ist und mit der Vorrichtung zur Erzeugung eines Überdrucks (7) und den Hilfsvorrichtungen (12) in Wirkverbindung steht, und

wobei der zweite Körper (2b) eine Innenwand (20) aufweist, die dem Gehäusesitz (14) zugewandt ist, und eine Außenwand (21), die nach außen gerichtet ist, wobei die elektronische Steuereinheit (19) der Innenwand (20) zugeordnet ist,

wobei die elektronische Steuereinheit (19) mit einer grafischen Schnittstellenanzeige (22) in Wirkverbindung steht, wobei der Mikroprozessor dazu ausgebildet ist, die zumindest von den Hilfsvorrichtungen (12) erfassten Signale zu empfangen und zu verarbeiten, um sie zumindest teilweise in grafischer und/oder numerischer Form auf der grafischen Schnittstellenanzeige (22) erscheinen zu lassen,

und wobei die Außenwand (21) aus der grafischen Schnittstellenanzeige (22) besteht, die eine Fläche der Vorrichtung (1) definiert.

2. Vorrichtung (1) nach Anspruch 1, **dadurch gekennzeichnet, dass** der Kreislauf (3) mindestens eine zusätzliche Vorrichtung zur Erzeugung eines Überdrucks umfasst, die lösbar direkt mit der Einlassmündung (5) für den Sauerstoff verbunden ist.

3. Vorrichtung (1) nach einem oder mehreren der vorhergehenden Ansprüche, wobei die Hilfsvorrichtungen (12) im Inneren des kastenförmigen Körpers (2) enthalten sind.

4. Vorrichtung (1) nach einem oder mehreren der vorhergehenden Ansprüche, wobei die Einlassöffnung (4) für die Luft und die Auslassöffnung (6) für ein Gemisch um einen Winkel zwischen 80° und 100° zueinander versetzt sind.

5. Vorrichtung (1) nach einem oder mehreren der Ansprüche 1 bis 3, wobei die Einlassöffnung (4) für die Luft und die Auslassöffnung (6) für ein Gemisch im Wesentlichen parallel zueinander sind.

6. Vorrichtung (1) nach einem oder mehreren der vorhergehenden Ansprüche, wobei sie mindestens ein Oximeter umfasst, das außerhalb des kastenförmigen Körpers (2) angeordnet ist und mit der elektronischen Steuereinheit (19) in Wirkverbindung steht, wobei das Oximeter dazu ausgebildet ist, die Blutsättigung (SpO$_2$) und die Herzfrequenz des Patienten zu erfassen.

7. Vorrichtung (1) nach Anspruch 6, wobei der Mikroprozessor mit Mitteln ausgestattet ist, die ausgebildet sind, um die Atemrate auf der Grundlage des Wertes des vom Durchflussmessgerät erfassten Durchflusses des Luft- und Sauerstoffgemisches zu berechnen und

wobei der Mikroprozessor mit Mitteln versehen ist, die dazu ausgebildet sind, auf der Grundlage der Werte der berechneten Herzfrequenz, des von dem Sauerstoffsensor erfassten Sauerstoffanteils (FiO$_2$) und der von dem Oximeter erfassten Blutsättigung (SpO$_2$) einen Indikator für die Wirksamkeit der Therapie bei dem Patienten (RO$_x$) zu berechnen.

8. Vorrichtung (1) nach Anspruch 7, wobei die elektronische Steuereinheit (19) mindestens einen Speicher umfasst, der mit mindestens einem Referenzwert des Indikators für die Wirksamkeit der Therapie am Patienten (RO$_x$) programmierbar ist, und der Mikroprozessor dazu ausgebildet ist, den berechneten Wert des Indikators für die Wirksamkeit der Therapie am Patienten mit dem Referenzwert zu vergleichen.

**Revendications**

1. - Dispositif (1) destiné à la ventilation pulmonaire, comprenant :

- un corps en forme de boîte (2) ;

- un circuit (3) reçu à l'intérieur dudit corps en forme de boîte (2) et comprenant au moins : un orifice d'entrée (4) pour l'air, une embouchure d'entrée (5) pour l'oxygène et un orifice de sortie (6) pour un mélange d'air et d'oxygène, un dispositif pour générer une pression positive (7) ;

dans lequel ledit circuit (3) comprend un corps monolithique rigide (8) comprenant au moins un canal principal (9), qui définit ledit orifice d'entrée (4) pour l'air et ledit orifice de sortie (6) pour un mélange, et au moins un canal secondaire (10), qui définit ladite embouchure d'entrée (5) pour l'oxygène et qui communique avec ledit canal principal (9), ladite embouchure d'entrée (5) étant apte à être reliée à une source d'oxygène sous pression et permettant ainsi à de l'oxygène sous pression d'être fourni le long dudit canal principal (9) afin de le mélanger avec l'air passant par le canal principal lui-même, et ledit corps monolithique rigide (8) définissant ledit canal principal (9) et définissant au moins en partie ledit canal secondaire (10),

ledit corps monolithique rigide (8) comprenant une première demi-coque (8a) et une seconde demi-coque (8b) verrouillées ensemble l'une à l'autre,

dans lequel ledit circuit (3) comprend une pluralité de dispositifs auxiliaires (12) choisis dans un groupe comprenant au moins un débitmètre qui est apte à détecter le débit du mélange d'air et d'oxygène s'écoulant par ledit canal principal (9), un capteur d'oxygène apte à détecter la fraction d'oxygène ($FiO_2$) présente dans le mélange d'air et d'oxygène s'écoulant par ledit canal principal (9), un capteur de pression apte à détecter la pression du mélange d'air et d'oxygène s'écoulant par ledit canal principal (9),

ledit corps monolithique rigide (8) comprenant une pluralité d'ouvertures prédéfinies (13) et lesdits dispositifs auxiliaires (12) étant associés auxdites ouvertures prédéfinies (13), lesdites ouvertures prédéfinies (13) communiquant avec au moins l'un dudit canal principal (9) et dudit canal secondaire (10),

dans lequel ledit corps en forme de boîte (2) comprend un premier corps (2a) définissant un siège de logement (14) dudit corps monolithique rigide (8) comportant au moins une ouverture d'accès (15) et comprend au moins un second corps (2b) associé d'une manière amovible audit premier corps (2a) pour la fermeture de ladite ouverture d'accès (15), et

dans lequel ledit dispositif comprend des moyens de raccordement amovible (16) dudit corps monolithique rigide (8) audit premier corps (2a),

dans lequel ledit dispositif pour générer une pression positive (7) est raccordé d'une manière actionnée par fluide audit corps monolithique rigide (8),

dans lequel ledit dispositif pour générer une pression positive (7) est directement associé d'une manière amovible audit orifice d'entrée (4) pour l'air,

dans lequel le dispositif (1) comprend au moins une unité de commande électronique (19) comportant un microprocesseur et reliée de manière fonctionnelle audit dispositif pour générer une pression positive (7) et auxdits dispositifs auxiliaires (12), et dans lequel ledit second corps (2b) a une paroi interne (20), orientée vers ledit siège de logement (14), et une paroi externe (21), orientée vers l'extérieur, ladite unité de commande électronique (19) étant associée à ladite paroi interne (20),

dans lequel ladite unité de commande électronique (19) est reliée de manière fonctionnelle à un dispositif d'affichage d'interface graphique (22), ledit microprocesseur étant apte à recevoir et traiter les signaux détectés au moins par lesdits dispositifs auxiliaires (12) afin de les faire apparaître au moins partiellement sous forme graphique et/ou numérique sur ledit dispositif d'affichage d'interface graphique (22), et

dans lequel ladite paroi externe (21) est composée dudit dispositif d'affichage d'interface graphique (22), qui définit une face du dispositif (1).

2. - Dispositif (1) selon la revendication 1, dans lequel ledit circuit (3) comprend au moins un dispositif supplémentaire pour la génération d'une pression positive directement associé d'une manière amovible à ladite embouchure d'entrée (5) pour l'oxygène.

3. - Dispositif (1) selon l'une ou plusieurs des revendications précédentes, dans lequel lesdits dispositifs auxiliaires (12) sont contenus à l'intérieur dudit corps en forme de boîte (2).

4. - Dispositif (1) selon l'une ou plusieurs des revendications précédentes, dans lequel ledit orifice d'entrée (4) pour l'air et ledit orifice de sortie (6) pour un mélange sont décalés l'un par rapport à l'autre d'un angle compris entre 80° et 100°.

5. - Dispositif (1) selon l'une ou plusieurs des revendications 1 à 3, dans lequel ledit orifice d'entrée (4) pour l'air et ledit orifice de sortie (6) pour un mélange sont sensiblement parallèles l'un à l'autre.

6. - Dispositif (1) selon l'une ou plusieurs des revendications précédentes, dans lequel il comprend au moins un oxymètre positionné à l'extérieur dudit corps en forme de boîte (2) et relié de manière fonctionnelle à ladite unité de commande électronique (19), ledit oxymètre étant apte à détecter la saturation sanguine ($SpO_2$) et la fréquence cardiaque du patient.

7. - Dispositif (1) selon la revendication 6, dans lequel ledit microprocesseur comporte des moyens aptes à calculer la fréquence respiratoire sur la base de la valeur du débit du mélange d'air et d'oxygène détecté par le débitmètre, et dans lequel ledit microprocesseur comporte des moyens aptes à calculer un indicateur de l'efficacité de la thérapie sur le patient ($RO_x$), sur la base des valeurs de la fréquence cardiaque calculée, de la fraction d'oxygène ($FiO_2$) détectée par ledit capteur d'oxygène et de la saturation sanguine ($SpO_2$) détectée par ledit oxymètre.

8. - Dispositif (1) selon la revendication 7, dans lequel ladite unité de commande électronique (19) comprend au moins une mémoire programmable avec au moins une valeur de référence de l'indicateur de l'efficacité de la thérapie sur le patient ($RO_x$) et le microprocesseur est configuré pour comparer la valeur calculée de l'indicateur de l'efficacité de la thérapie sur le patient à ladite valeur de référence.

Fig.1

Fig.2

Fig.3

Fig.4

**Fig.5**

**Fig.6**

Fig.7

**EP 4 192 558 B1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2007227360 A1
- WO 2013038342 A1
- WO 2019070136 A1
- WO 03064009 A1
- US 2015250962 A1
- US 2015144136 A1